# EUROPEAN PATENT SPECIFICATION

(11) **EP 2 484 361 B1**
(45) Date of publication and mention of the grant of the patent: **11.11.2015**
(21) Application number: 10818895.4
(22) Date of filing: 27.09.2010
(51) Int. Cl.: A61K 31/423, A61P 3/04, A61P 3/06, A61P 9/00, A61P 9/10, A61P 13/12, A61P 29/00, A61P 43/00, C07D 263/58

(54) **AGENT FOR REDUCING VISCERAL FAT WEIGHT**
MITTEL ZUR REDUZIERUNG DES GEWICHTS VON VISZERALEM FETT
AGENT POUR RÉDUIRE LE POIDS DE GRAISSE VISCÉRALE

(30) Priority: 28.09.2009 JP 2009222853
(43) Date of publication of application: 08.08.2012
(73) Proprietor: Kowa Company, Ltd., Nagoya-shi, Aichi 460-8625 (JP)
(72) Inventor: TAKIZAWA, Toshiaki, Higashimurayama-shi Tokyo 189-0022 (JP); MURAKAMI, Kentaro, Higashimurayama-shi Tokyo 189-0022 (JP)
(74) Representative: Blodig, Wolfgang
(86) International application number: PCT/JP2010/066678
(87) International publication number: WO 2011/037223

(56) References cited:
- EP-A1- 1 661 890
- EP-A1- 2 141 155
- WO-A1-97/36579
- WO-A1-2005/023777
- WO-A1-2008/120472
- YAMAZAKI ET AL: "Design and synthesis of highly potent and selective human peroxisome proliferator-activated receptor alpha agonists", BIOORGANIC & MEDICINAL CHEMISTRY LETTERS, PERGAMON, ELSEVIER SCIENCE, GB, vol. 17, no. 16, 17 July 2007 (2007-07-17) , pages 4689-4693, XP022181890, ISSN: 0960-894X, DOI: 10.1016/J.BMCL.2007.05.066
- MICHAEL K. BADMAN ET AL.: 'Hepatic Fibroblast Growth Factor 21 Is Regulatedby PPARa and Is a Key Mediatorof Hepatic Lipid Metabolism in Ketotic States' CELL METABOLISM vol. 5, 2007, pages 426 - 437, XP008151941
- CECILIA GALMAN ET AL.: 'The Circulating Metabolic RegulatorFGF21 Is Induced by Prolonged Fastingand PPARa Activation in Man' CELL METABOLISM vol. 8, 2008, pages 169 - 174, XP008151936
- TAKESHI INAGAKI ET AL.: 'Endocrine Regulation of the Fasting Responseby PPARa-Mediated Inductionof Fibroblast Growth Factor 21' CELL METABOLISM vol. 5, 2007, pages 415 - 425, XP008151942
- THOMAS LUNDASEN ET AL.: 'PPARa is a key regulator of hepatic FGF21' BIOCHEMICAL AND BIOPHYSICAL RESEARCH COMMUNICATIONS vol. 360, no. 2, 2007, pages 437 - 440, XP022145176
- SADAO TAKAHASHI ET AL.: 'Metabolic Syndrome no Kagi Bunshi PPARs' CARDIOANGIOLOGY vol. 59, no. 2, 2006, pages 160 - 167, XP008153472
- SHIZUYA YAMASHITA: 'Metabolic Syndrome Kenkyu no Shinten Metabolic Syndrome no Chiryo Senryaku no Shinpo' NAIBUNPITSU-JUNKANKIKA vol. 24, no. 5, 2007, pages 484 - 490, XP008151955
- TOSHIMASA YAMAUCHI ET AL.: 'Naizo Himan ni Taisuru Yakubutsu Chiryo' BESSATSU-IGAKU NO AYUMI NAFLD NO SUBETE 05 March 2006, pages 144 - 149, XP008153469
- MOTOKO UCHIYAMA ET AL.: 'Ko Himan Koka Hyokakei no Kento - Ko Shiboshoku Sesshu Oyobi PPARa Agonist ga KK-Ay Mouse ni Ataeru Eikyo' DAI 61 KAI THE JAPANESE SOCIETY OF NUTRITION AND FOOD SCIENCE TAIKAI KOEN YOSHISHU 20 April 2007, page 204, XP008151954
- MASAKI TSUNODA ET AL.: 'A novel PPARa agonist ameliorates insulin resistance in dogs feda high-fat diet' AMERICAN JOURNAL OF PHYSIOLOGY. ENDOCRINOLOGY AND METABOLISM vol. 294, 2008, pages E833 - E840, XP008151952
- IKUO IKEDA ET AL.: 'Campest-5-en-3-one, an oxidized derivative of campesterol, activates PPARa, promotes energy consumption and reduces visceral fat deposition in rats' BIOCHIMICA ET BIOPHYSICA ACTA vol. 1760, 2006, pages 800 - 807, XP025015031
- HUN CS, HASEGAWA K, KAWABATA T, KATO M, SHIMOKAWA T, KAGAWA Y.: "Increased uncoupling protein2 mRNA in white adipose tissue, and decrease in leptin, visceral fat, blood glucose, and cholesterol in KK-Ay mice fed with eicosapentaenoic and docosahexaenoic acids in addition to linolenic acid.", BIOCHEM BIOPHYS RES COMMUN., 27 May 1999 (1999-05-27), pages 85-90,
- CHEN W, ZHOU XB, LIU HY, XU C, WANG LL, LI S.: "P633H, a novel dual agonist at peroxisome proliferator-activated receptors alpha and gamma, with different anti-diabetic effects in db/db and KK-Ay mice.", BR J PHARMACOL., 1 July 2009 (2009-07-01), pages 724-735,
- AGUIRRE L, HIJONA E, MACARULLA MT, GRACIA A, LARRECHI I, BUJANDA L, HIJONA L, PORTILLO MP.: "Several statins increase body and liver fat accumulation in a model of metabolic syndrome.", , 1 June 2013 (2013-06-01), pages 281-288,

## Description

### [Field of the Invention]

The present invention relates to an agent for promoting production of FGF21 (hereinafter may be referred to as an "FGF21 production promoting agent"), an agent for reducing visceral fat weight (hereinafter may be referred to as a "visceral fat weight reducing agent"), and a preventive and/or therapeutic agent for visceral fat obesity, each of the agents containing, as an active ingredient, a compound of claim 1 which selectively activates α-type peroxisome proliferator-activated receptor (PPARα).

### [Background of the Invention]

PPAR is a receptor belonging to the nuclear receptor family. PPAR binds to specific sites (peroxisome proliferator response elements, PPREs) of a target gene, and positively or negatively regulates transcription of the gene. As has been known, this receptor has three subtypes (α, y, and δ) (Non-Patent Document 1).

Of these subtypes, PPARα is mainly expressed in the liver. When PPARα is activated, production of apoC-III, which is a lipoprotein lipase (LPL)-inhibiting protein, is suppressed, and subsequently LPL is activated, resulting in lipolysis.

Hitherto known PPARα agonists include unsaturated fatty acids, and fibrate drugs such as fenofibrate, bezafibrate, and gemfibrozil (Non-Patent Document 2). As has also been reported, such a PPARα agonist activates acetyl-CoA carboxylase (ACC) or carnitine palmitoyltransferase 1 (CPT-1), which is involved in β-oxidation of a fatty acid.

Meanwhile, hitherto, 22 fibroblast growth factors (FGFs) have been shown to be present in human, on the basis of amino acid sequence homology (Patent Document 1). As has been known, such an FGF not only acts on growth of fibroblasts, but also is deeply involved in a variety of biological phenomena, including morphogenesis, angiogenesis, tumorigenesis, wound healing, maintenance of neuronal survival, and metabolic regulation (Non-Patent Documents 3 and 4). In association with identification of human FGF21 by Nishimura, *et al.* (Non-Patent Document 5), FGF21 has been reported to promote lipolysis, to suppress fat accumulation, and to ameliorate hypercholesterolemia, diabetes (hyperglycemia, insulin resistance), or obesity (Non-Patent Document 6).

Ito, *et al.* inhibited the function of FGF21 in zebrafish and analyzed the resultant phenotype. As a result, they found that erythrocytes are lost or decreased in FGF21-inhibited zebrafish, and that loss or decrease of erythrocytes is attributed to inhibition of differentiation of hematopoietic stem cells into erythroid-myeloid cells (Non-Patent Document 7). They also found that inhibition of the function of FGF21 in zebrafish can promote differentiation of hematopoietic stem cells into lymphoid cells.

Thus, FGF21 is considered to be capable of controlling differentiation of hematopoietic stem cells into erythroid-myeloid cells (e.g., erythrocytes, megakaryocytes, eosinophils, neutrophils, basophils, monocytes, and dendritic cells) or into lymphoid cells (e.g., T cells, B cells, and NK cells). As has been reported, a substance which regulates the expression or function of FGF21 has the ability to regulate differentiation of hematopoietic stem cells into erythroid-myeloid cells or into lymphoid cells, and the substance is useful for, for example, development of a laboratory reagent or a drug for a disease caused by abnormal blood cells (e.g., hematopoietic disease, immune disease, or allergic disease) (Patent Document 1).

Fenofibrate, which is a PPARα agonist, has been known as a substance which promotes production of FGF21. As has been reported, when a fenofibrate drug is administered to a hypertriglyceridemia patient for two weeks, the serum FGF21 level increases by 28% (Non-Patent Document 8). In order to cause fenofibrate to exhibit the effect of reducing visceral fat weight, administration of a higher dose of fenofibrate would be required. However, as has been reported, when a high dose of fenofibrate is administered to a rat model, adverse events (e.g., decrease in hemoglobin, or necrosis of liver cells) occur in the model (Non-Patent Document 9). Thus, a limitation is imposed on the high-dose administration of fenofibrate. In fact, no clinical studies have reported that administration of fenofibrate reduces visceral fat weight.

In recent years, there have been reported compounds which potently and selectively activate PPARα, as compared with fibrate drugs (Patent Documents 2 to 11). However, it has not been reported that such a compound promotes production of FGF21, nor that the compound exhibits the effect of reducing visceral fat weight.

Hitherto, methods known in the art for markedly increasing blood FGF21 level include only injection of FGF21 protein (Non-Patent Document 10), incorporation of the FGF21 gene (Non-Patent Document 11), and activation of CREB-H (i.e., another transcription factor which promotes expression of FGF21) through gene transfer (Non-Patent Document 12). However, the safety of such a method has not yet been established. Particularly, when incorporation of the FGF21 gene or the CREB-H gene, which requires employment of a vector, is applied to human, adverse side effects are considered to occur with high probability.

Types of obesity include subcutaneous fat obesity (subcutaneous accumulation of fat), visceral fat obesity (accumulation of fat around internal organs), and hidden obesity (normal physical appearance but high body fat percentage). Particularly, visceral fat obesity may cause metabolic syndrome, and may involve a risk of developing serious diseases. Therefore, a compound exhibiting the effect of reducing visceral fat weight may be useful as a preventive or therapeutic agent for obesity (in particular, visceral fat obesity) or metabolic syndrome. Thus, demand has arisen for a visceral fat weight reducing agent which has no side effects.

### Prior Art Document

### Patent Document

Patent Document 1: Japanese Patent Application Laid-Open (*kokai*) No. 2006-246823
Patent Document 2: WO 2005/023777
Patent Document 3: WO 2009/080248
Patent Document 4: WO 2009/047240
Patent Document 5: WO 2008/006043
Patent Document 6: WO 2006/049232
Patent Document 7: WO 2006/033891
Patent Document 8: WO 2005/009942
Patent Document 9: WO 2004/103997
Patent Document 10: WO 2005/097784
Patent Document 11: WO 2003/043997

### Non-Patent Document

Non-Patent Document 1: J. Lipid Research 37, 907-925 (1996)
Non-Patent Document 2: Trends in Endoclinology Metabolism 15, 324-330 (2004)
Non-Patent Document 3: Genome Biol. 21, REVIEW, 3005 (2001)
Non-Patent Document 4: Trends Genet. 20, 563-569 (2004)
Non-Patent Document 5: Biochim. Biophys. Acta, 1492, 203-206 (2000)
Non-Patent Document 6: Biodrugs, 22 (1), 37-44 (2008)
Non-Patent Document 7: EMBO Rep. 7, 649-54 (2006)
Non-Patent Document 8: Cell Metabolism, 8, 169-174 (2008)
Non-Patent Document 9: J. Pharmacol. Ther. 23, 15-36 (1995)
Non-Patent Document 10: Diabetes, 58, 250-259 (2009)
Non-Patent Document 11: J. Clin. Invest. 115, 1627-1635 (2005)
Non-Patent Document 12: Diabetes 52 (Suppl. 1), S-157 (2009)

The 24th Annual Meeting of Japan Diabetes Society, Abstract, 2009

### [Summary of the Invention]

### [Problems to be Solved by the Invention]

An object of the present invention is to provide a drug which has few side effects, which promotes production of FGF21, which reduces visceral fat weight, and which is useful for prevention and/or treatment of visceral fat obesity.

### [Means for Solving the Problems]

In view of the foregoing, the present inventors have conducted extensive studies, and as a result have found that a phenoxyacetic acid derivative represented by the below-described formula (1) or a salt thereof which is disclosed in Patent Document 2, and is shown to selectively activate PPARα and to be useful as a preventive and/or therapeutic drug for, for example, hyperlipidemia, arteriosclerosis, diabetes, diabetic complications (e.g., diabetic nephropathy), inflammation, or cardiac diseases in mammals (including human) without causing an increase in body weight or obesity significantly increases plasma FGF21 level and exhibits an excellent effect of reducing visceral fat weight. The present invention has been accomplished on the basis of this finding.

The present invention relates to a compound selected from (R)-2-[3-[[N-(benzoxazol-2-yl)-N-3-(4-methoxyphenoxy)propyl]aminomethyl]phenoxy]butyric acid and (R)-2-[3-[[N-(5-fluorobenzoxazol-2-yl)-N-2-phenoxyethyl]aminomethyl]phenoxy]butyric acid, a salt thereof, or a solvate of the compound or the salt, for use in prevention and/or treatment of visceral fat obesity as well as to a pharmaceutical composition for use in a method of treatment and/or prevention of visceral fat obesity, the composition comprising a compound selected from (R)-2-[3-[[N-(benzoxazol-2-yl)-N-3-(4-methoxyphenoxy)propyl]aminomethyl]-phenoxy]butyric acid and (R)-2-[3-[[N-(5-fluorobenzoxazol-2-yl)-N-2-phenoxyethyl]aminomethyl]phenoxy]butyric acid, a salt thereof, or a solvate of the compound or the salt.

Accordingly, the present disclosure provides an FGF21 production promoting agent containing, as an active ingredient, a compound represented by the following formula (1) :

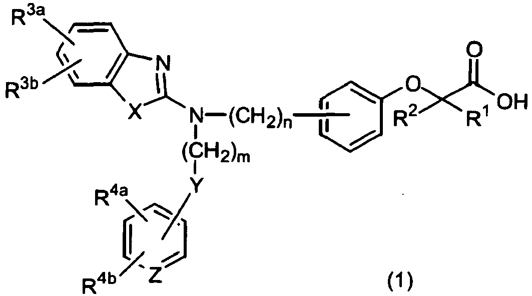

(wherein each of R¹ and R², which may be identical to or different from each other, represents a hydrogen atom, a methyl group, or an ethyl group; each of R^{3a}, R^{3b}, R^{4a}, and R^{4b}, which may be identical to or different from one another, represents a hydrogen atom, a halogen atom, a nitro group, a hydroxyl group, a C₁₋₄ alkyl group, a trifluoromethyl group, a C₁₋₄ alkoxy group, a C₁₋₄ alkylcarbonyloxy group, a di-C₁₋₄ alkylamino group, a C₁₋₄ alkylsulfonyloxy group, a C₁₋₄ alkylsulfonyl group, a C₁₋₄ alkylsulfinyl group, or a C₁₋₄ alkylthio group, or R^{3a} and R^{3b} or R^{4a} and R^{4b} may bond together to form an alkylenedioxy group; X represents an oxygen atom, a sulfur atom, or N-R⁵ (R⁵ represents a hydrogen atom, a C₁₋₄ alkyl group, a C₁₋₄ alkylsulfonyl group, or a C₁₋₄ alkyloxycarbonyl group); Y represents an oxygen atom, an S(O)ₗ group (1 is a number of from 0 to 2), a carbonyl group, a carbonylamino group, an aminocarbonyl group, a sulfonylamino group, an aminosulfonyl group, or an NH group; Z represents CH or N; n is a number of from 1 to 6; and m is a number of from 2 to 6), a salt of the compound, or a solvate of the compound or the salt.

The present disclosure also provides a visceral fat weight reducing agent containing, as an active ingredient, a compound represented by formula (1), a salt of the compound, or a solvate of the compound or the salt.

The present disclosure also provides a preventive and/or therapeutic agent for obesity containing, as an active ingredient, a compound represented by formula (1), a salt of the compound, or a solvate of the compound or the salt. More specifically, the present disclosure provides a preventive and/or therapeutic agent for visceral fat obesity containing, as an active ingredient, a compound represented by formula (1), a salt of the compound, or a solvate of the compound or the salt.

The present disclosure also provides a preventive and/or therapeutic agent for metabolic syndrome containing, as an active ingredient, a compound represented by formula (1), a salt of the compound, or a solvate of the compound or the salt.

The present disclosure also provides a method for use in promoting production of FGF21, characterized by administering, to a subject in need thereof, an effective amount of a compound represented by formula (1), a salt of the compound, or a solvate of the compound or the salt.

The present disclosure also provides a method for use in reducing visceral fat weight, characterized by administering, to a subject in need thereof, an effective amount of a compound represented by formula (1), a salt of the compound, or a solvate of the compound or the salt.

The present disclosure also provides a method for use in prevention and/or treatment of obesity, characterized by administering, to a subject in need thereof, an effective amount of a compound represented by formula (1), a salt of the compound, or a solvate of the compound or the salt. More specifically, the present disclosure provides a method for use in prevention and/or treatment of visceral fat obesity, characterized by administering, to a subject in need thereof, an effective amount of a compound represented by formula (1), a salt of the compound, or a solvate of the compound or the salt.

The present disclosure also provides a method for use in prevention and/or treatment of metabolic syndrome, characterized by administering, to a subject in need thereof, an effective amount of a compound represented by formula (1), a salt of the compound, or a solvate of the compound or the salt.

The present disclosure also provides use of a compound represented by formula (1), a salt of the compound, or a solvate of the compound or the salt in the production of an FGF21 production promoting agent.

The present disclosure also provides use of a compound represented by formula (1), a salt of the compound, or a solvate of the compound or the salt in the production of a visceral fat weight reducing agent.

The present disclosure also provides use of a compound represented by formula (1), a salt of the compound, or a solvate of the compound or the salt in the production of a preventive and/or therapeutic agent for obesity. More specifically, the present disclosure provides use of a compound represented by formula (1), a salt of the compound, or a solvate of the compound or the salt in the production of a preventive and/or therapeutic agent for visceral fat obesity.

The present disclosure also provides use of a compound represented by formula (1), a salt of the compound, or a solvate of the compound or the salt in the production of a preventive and/or therapeutic agent for metabolic syndrome. The present disclosure also provides a compound represented by formula (1), a salt of the compound, or a solvate of the compound or the salt, for promoting FGF21 production.

The present disclosure also provides a compound represented by formula (1), a salt of the compound, or a solvate of the compound or the salt, for reduction of visceral fat weight.

The present disclosure also provides a compound represented by formula (1), a salt of the compound, or a solvate of the compound or the salt, for prevention and/or treatment of obesity (more specifically, visceral fat obesity).

The present disclosure also provides a compound represented by formula (1), a salt of the compound, or a solvate of the compound or the salt, for prevention and/or treatment of metabolic syndrome.

### [Effects of the Invention]

The drug of the present invention exhibits excellent effects of promoting production of FGF21 and reducing visceral fat weight, as well as high safety. Therefore, the drug is useful for prevention and/or treatment of visceral fat obesity

### [Brief Description of the Drawings]

[Fig. 1] Fig. 1 shows the effect of compound A or fenofibrate on plasma triglyceride (TG) level.
[Fig. 2] Fig. 2 shows the effect of compound A or fenofibrate on plasma FGF21 level.
[Fig. 3] Fig. 3 shows the correlation between plasma FGF21 level and plasma triglyceride (TG) level in the case of administration of compound A or fenofibrate.
[Fig. 4] Fig. 4 shows the effect of compound A or fenofibrate on epididymal fat weight.

### [Modes for Carrying Out the Invention]

Examples of the halogen atom represented by R^{3a}, R^{3b}, R^{4a}, or R^{4b} in formula (1) include a fluorine atom, a chlorine atom, and a bromine atom. Of these, a fluorine atom and a chlorine atom are particularly preferred.

Examples of the C₁₋₄ alkyl group represented by R^{3a}, R^{3b}, R^{4a}, R^{4b}, or R⁵ include a methyl group, an ethyl group, an n-propyl group, an isopropyl group, and a butyl group. Of these, a methyl group is particularly preferred.

Examples of the C₁₋₄ alkoxy group represented by R^{3a}, R^{3b}, R^{4a}, or R^{4b} include a methoxy group, an ethoxy group, an n-propoxy group, an isopropoxy group, and a butoxy group. Of these, a methoxy group is particularly preferred.

Examples of the C₁₋₄ alkylcarbonyloxy group represented by R^{3a}, R^{3b}, R^{4a}, or R^{4b} include a methylcarbonyloxy group, an ethylcarbonyloxy group, an n-propylcarbonyloxy group, an isopropylcarbonyloxy group, and a butylcarbonyloxy group. Of these, a methylcarbonyloxy group is particularly preferred.

Examples of the di-C₁₋₄ alkylamino group represented by R^{3a}, R^{3b}, R^{4a}, or R^{4b} include a dimethylamino group, a diethylamino group, and a diisopropylamino group. Of these, a dimethylamino group is particularly preferred.

Examples of the C₁₋₄ alkylsulfonyloxy group represented by R^{3a}, R^{3b}, R^{4a}, or R^{4b} include a methylsulfonyloxy group and an ethylsulfonyloxy group. Of these, a methylsulfonyloxy group is particularly preferred.

Examples of the C₁₋₄ alkylsulfonyl group represented by R^{3a}, R^{3b}, R^{4a}, R^{4b}, or R⁵ include a methylsulfonyl group and an ethylsulfonyl group. Of these, a methylsulfonyl group is particularly preferred.

Examples of the C₁₋₄ alkylsulfinyl group represented by R^{3a}, R^{3b}, R^{4a}, or R^{4b} include a methylsulfinyl group and an ethylsulfinyl group. Of these, a methylsulfinyl group is particularly preferred.

Examples of the C₁₋₄ alkylthio group represented by R^{3a}, R^{3b}, R^{4a}, or R^{4b} include a methylthio group and an ethylthio group. Of these, a methylthio group is particularly preferred.

Examples of the alkylenedioxy group formed through bonding of R^{3a} and R^{3b} or bonding of R^{4a} and R^{4b} include a methylenedioxy group and an ethylenedioxy group. Of these, a methylenedioxy group is particularly preferred.

Examples of the C₁₋₄ alkyloxycarbonyl group represented by R⁵ include a methyloxycarbonyl group and an ethyloxycarbonyl group. Of these, a methyloxycarbonyl group is particularly preferred.

It is particularly preferred when both R¹ and R² are hydrogen atoms or methyl groups, or when R¹ and R² are a methyl group and a hydrogen atom, respectively, or are an ethyl group and a hydrogen atom, respectively.

X represents an oxygen atom, a sulfur atom, or N-R⁵, and is preferably an oxygen atom. Y represents an oxygen atom, S(O)ₗ, a carbonyl group, a carbonylamino group, an aminocarbonyl group, a sulfonylamino group, an aminosulfonyl group, or an NH group, and is preferably an oxygen atom. Z represents CH or N, and is preferably CH. In formula (1), 1 is a number of from 0 to 2 (preferably 2); n is a number of from 1 to 6 (preferably a number of from 1 to 3); and m is a number of from 2 to 6 (preferably 2 to 4, particularly preferably 2 or 3).

The compounds of the present invention are (R)-2-[3-[[N-(benzoxazol-2-yl)-N-3-(4-methoxyphenoxy)propyl]aminomethyl]phenoxy]butyric acid or (R)-2-[3-[[N-(5-fluorobenzoxazol-2-yl)-N-2-phenoxyethyl]aminomethyl]phenoxy]butyric acid.

The compound of the present disclosure represented by formula (1) as well as the compounds of claim 1 may be produced through, for example, the method described in WO 2005/023777.

The present invention may employ a salt or solvate of the compound. The salt or the solvate may be produced through a customary method.

No particular limitation is imposed on the salt of the compound of the present invention so long as it is a pharmaceutically acceptable one. Examples of the salt include alkali metal salts such as sodium salt and potassium salt; alkaline earth metal salts such as calcium salt and magnesium salt; organic base salts such as ammonium salt and trialkylamine salt; mineral acid salts such as hydrochloride and sulfate; and organic acid salts such as acetate.

Examples of the solvate of the compound of the present invention or the salt of the compound include a hydrate and an alcohol solvate (e.g., ethanol solvate).

The compound of the present disclosure represented by formula (1) has an asymmetric carbon atom, and thus has optical isomers (R-isomer and S-isomer). The present invention encompasses all the optical isomers.

As described in the Examples hereinbelow, the compound of the invention exhibited the effect of markedly reducing plasma triglyceride level and the effect of significantly increasing plasma FGF21 level in an evaluation system employing KK-Ay mice or hyperlipidemic Zucker fatty rats with obesity due to overeating. In addition, the compound significantly reduced visceral fat weight. Therefore, the drug of the present invention is useful for prevention and/or treatment of visceral fat obesity.

The compound of the present invention , a salt of the compound, or a solvate of the compound or the salt may be prepared, by itself or in combination with a pharmaceutically acceptable carrier, into a variety of dosage forms, including tablet, capsule, granule, powder, lotion, ointment, injection, and suppository. Such a drug product may be produced through any known method. For example, when the compound is provided in the form of a peroral product, the product may be produced by appropriately formulating the compound in combination with, for example, a solubilizer (e.g., tragacanth gum, gum arabic, sucrose fatty acid ester, lecithin, olive oil, soybean oil, or PEG 400), an excipient (e.g., starch, mannitol, or lactose), a binder (e.g., carboxymethylcellulose sodium or hydroxypropylcellulose), a disintegrant (e.g., crystalline cellulose or carboxymethylcellulose calcium), a lubricant (e.g., talc or magnesium stearate), or a fluidity-improving agent (e.g., light silicic anhydride).

The compound of the present invention, a salt of the compound, or a solvate of the compound or the salt is orally or parenterally administered. The dose of the drug of the present invention may vary with the body weight, age, sex, symptom, etc. of a patient in need thereof. Generally, the daily dose of compound (1) for an adult is 0.01 to 1,000 mg, preferably 0.1 to 100 mg. Preferably, the daily dose is administered in a divided manner (once to thrice a day).

### [Examples]

The present invention will next be described in more detail by way of examples, which should not be construed as limiting the invention thereto.

### Example 1 Effects on Zucker fatty rat

This test employed (R)-2-[3-[[N-(benzoxazol-2-yl)-N-3-(4-methoxyphenoxy)propyl]aminomethyl]phenoxy]butyric acid (hereinafter will be referred to as "compound A"), which is an optically active substance of the compound disclosed in Example 14 in Patent Document 2. Compound A or fenofibrate was administered to Zucker fatty rats, and the effects of the compound on plasma triglyceride (TG) level, plasma FGF21 level, and epididymal fat weight were determined through the below-described methods.

### 1. Test animal and rearing environment

Hyperlipidemic Zucker fatty rats with obesity due to overeating (Crlj: ZUC-Lepr<fa> Genotype: fa/fa) and Zucker lean rats without obesity (Crlj: ZUC-Lepr<fa> Genotype: fa/+ or +/+) were purchased from Charles River Laboratories Japan Inc. (eight weeks old upon test).

Throughout the test period, the rats were reared in a rearing cage (light-dark cycle (light period by interior lighting: 7:00 a.m. to 7:00 p.m.), temperature: 23 ± 3°C, humidity: 55 ± 15%) under conditions where solid feed (CE-2, product of Oriental Yeast Co., Ltd.) and tap water were fed ad libitum.

### 2. Drug preparation

Each of compound A and fenofibrate was suspended in a 0.5 mass% aqueous solution of methylcellulose (Metolose (registered trademark), SM-400, product of Shin-Etsu Chemical Co., Ltd.) so that the dose of the aqueous solution was adjusted to 2 mL/kg. The resultant suspension was refrigerated in a light-shielding bottle at 4°C, and drug preparation was carried out every seven days.

### 3. Test method

Rats were divided into the following four groups (six rats per group) so that body weights, plasma total cholesterol levels, and plasma triglyceride levels were averaged in the groups: a first group (control), a second group (administration of compound A (1 mg/kg)), a third group (administration of compound A (3 mg/kg)), and a fourth group (administration of fenofibrate (300 mg/kg)). A group of five Zucker lean rats was employed as a negative control group.

Each drug was orally administered once a day (in the morning) for continuous 14 days. A 0.5 mass% aqueous methylcellulose solution (2 mL/kg) was orally administered to the rats of the first group (control).

On day 14 after initiation of administration, blood was collected after four-hour fasting following administration, and plasma triglyceride level and plasma FGF21 level were measured through an enzymatic method and ELISA (Human FGF-21 ELISA Kit, product of Biovender), respectively. On the day following blood collection, laparotomy was performed under anesthesia, and then epididymal fat was removed and weighed. Epididymal fat weight is highly correlated with total visceral fat weight, and thus is employed as an indicator for evaluating the effect of a drug on visceral fat weight (FASEB J. 20, 1203-1205 (2006)).

### 4. Statistical analysis and data processing method

The results were represented as mean values ± standard deviation. Comparison between the control group and the Zucker lean rat group was carried out by means of unpaired Student's t-test, and comparison between the control group and each drug administration group was carried out by means of Dunnett's multiple comparison test or Tukey's multiple comparison test (significant difference was considered for p < 0.05).

### 5. Test results

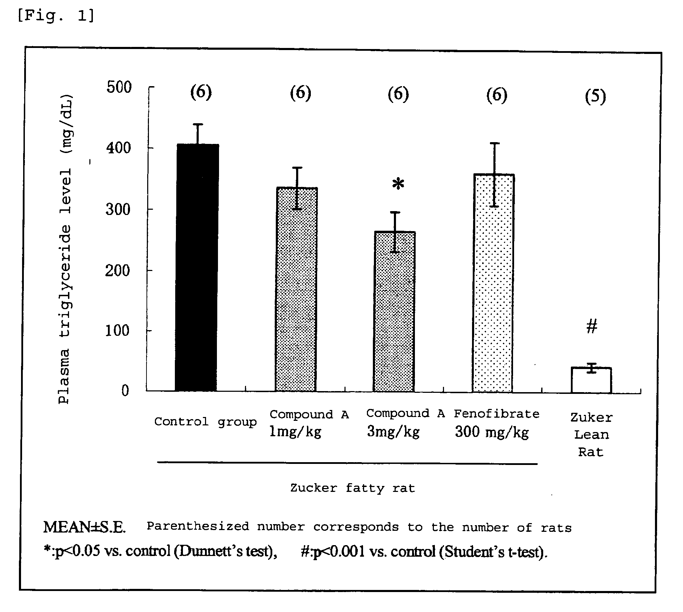

Fig. 1 shows data of plasma triglyceride level. Data on the horizontal axis correspond, from left to right, to the first group (control), the second group (administration of compound A (1 mg/kg)), the third group (administration of compound A (3 mg/kg)), the fourth group (administration of fenofibrate (300 mg/kg)), and the negative control group (Zucker lean rat). In Fig. 1, the symbol "*" represents a significant difference (p < 0.05), and the symbol "#" represents a significant difference (p < 0.001).

As shown in Fig. 1, the plasma triglyceride level in the third group was significantly (p < 0.05) lower than that in the control group; i.e., compound A clearly exhibited the effect of reducing plasma triglyceride level. In contrast, the plasma triglyceride level in the fourth group was only slightly lower than that in the control group.

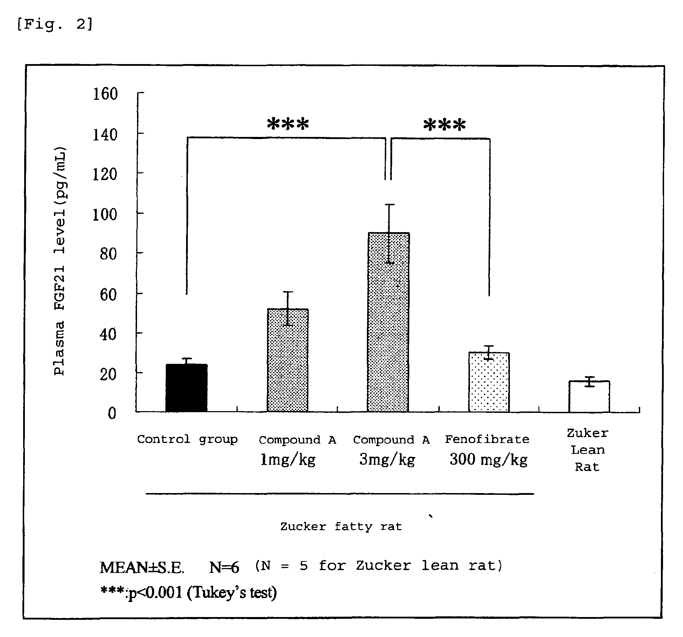

Fig. 2 shows data of plasma FGF21 level. Data on the horizontal axis correspond, from left to right, to the first group (control), the second group (administration of compound A (1 mg/kg)), the third group (administration of compound A (3 mg/kg)), the fourth group (administration of fenofibrate (300 mg/kg)), and the negative control group (Zucker lean rat). In Fig. 2, the symbol "***" represents a significant difference (p < 0.001).

As shown in Fig. 2, the plasma FGF21 level in the third group was significantly (p < 0.001) higher than that in the control group; i.e., compound A clearly exhibited the effect of promoting production of plasma FGF21. In contrast, the plasma FGF21 level in the fourth group was only slightly higher than that in the control group (P = 0.2).

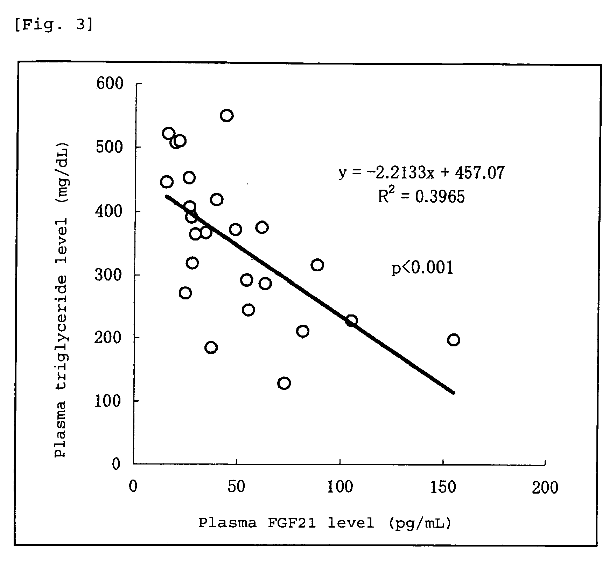

Fig. 3 shows the correlation between plasma triglyceride level and plasma FGF21 level. As shown in Fig. 3, a significant negative correlation is observed between them.

The aforementioned data indicate that, in the present animal model, compound A exhibits the effect of reducing plasma TG level and the effect of markedly enhancing expression of FGF21 (i.e., a new glucose/lipid metabolism-improving factor), and suggest that compound A can ameliorate lipid metabolism even in a pathological condition with highly abnormal lipid metabolism, by increasing plasma FGF21 level therein.

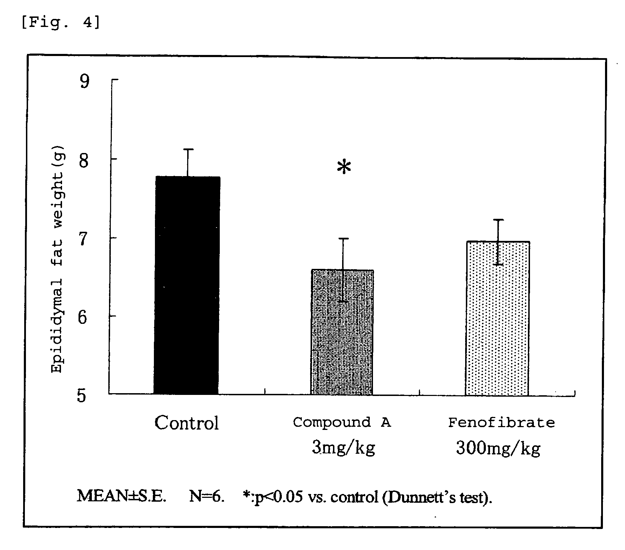

Fig. 4 shows the results of measurement of epididymal fat weight, which is an indicator of visceral fat weight. As shown in Fig. 4, the epididymal fat weight in the group of compound A administration (3 mg/kg) was considerably and statistically significantly lower than that in the control group (*: p < 0.05). During the period of compound A administration, adverse events were not particularly observed. In contrast, fenofibrate exhibited only a slight effect on epididymal fat weight, and a statistically significant difference was not observed between the epididymal fat weight in the group of fenofibrate administration and that in the control group. As has been reported, when fenofibrate is administered to rats at a dose of 30 mg/kg or more, various adverse events occur in the rats (Non-Patent Document 9). In the rats of the group of fenofibrate administration (300 mg/kg), feed intake was maintained equal to that in the rats of the control group for 10 days after administration. However, from 11 days to 14 days after administration, feed intake was reduced by 14% in the rats of the group of fenofibrate administration, which raised a concern about the toxicity of fenofibrate. Even under such a situation, fat weight tended to decrease only slightly.

The aforementioned data indicate that compound A markedly enhances production of FGF21 and thus potently reduces visceral fat weight. Also, the data indicate that these effects of compound A were clearly higher than those of fenofibrate (i.e., an existing fibrate and the most excellent PPARα agonist), and that compound A exhibits high safety.

### Example 2 Visceral fat weight reducing effect on KK-Ay mice

This test employed compound A, as well as (R)-2-[3-[[N-(5-fluorobenzoxazol-2-yl)-N-2-phenoxyethyl]aminomethyl]phenoxy]butyric acid (hereinafter will be referred to as "compound B"), which is an optically active substance of the compound disclosed in Example 229 in Patent Document 2. Compound A, compound B, or fenofibrate was administered to KK-Ay mice, and the effects of the compound on epididymal fat weight and mesenteric fat weight were determined through the below-described methods.

### 1. Test animal and rearing environment

Method: Male KK-Ay mice (KK-Ay/TaJcl) were purchased from Clea Japan, Inc. (10 weeks old upon test). Each compound was orally administered to mice once a day for four weeks. Throughout the test period, the mice were reared in a rearing cage (light-dark cycle (light period by interior lighting: 7:00 a.m. to 7:00 p.m.), temperature: 23 ± 3°C, humidity: 55 ± 15%) under conditions where solid feed (CE-2, product of Oriental Yeast Co., Ltd.) and tap water were fed ad libitum.

### 2. Drug preparation

Each of compound A, compound B, and fenofibrate was suspended in a 0.5 mass% aqueous solution of methylcellulose (Metolose (registered trademark), SM-400, product of Shin-Etsu Chemical Co., Ltd.) so that the dose of the aqueous solution was adjusted to 5 mL/kg. The resultant suspension was refrigerated in a light-shielding bottle at 4°C, and drug preparation was carried out every seven days.

### 3. Test method

Animals were divided into the following four groups (six animals per group): a first group (control), a second group (administration of compound A (1 mg/kg)), a third group (administration of compound B (3 mg/kg)), and a fourth group (administration of fenofibrate (300 mg/kg)).

Each drug was orally administered once a day (in the morning) for 35 days. A 0.5 mass% aqueous methylcellulose solution was orally administered (5 mL/kg) to the animals of the first group (control).

After completion of administration, epididymal fat and mesenteric fat were removed and weighed.

### 4. Statistical analysis and data processing method

The results were represented as percent reduction (mean value) in fat weight in each compound administration group with respect to the control group.

### 5. Test results

Table 1 shows the results. In the group of compound A administration (1 mg/kg) or the group of compound B administration (3 mg/kg), a more potent fat weight reducing effect was observed, as compared with the group of fenofibrate administration (300 mg/kg). During the period of compound A or compound B administration, adverse events were not particularly observed.

**[Table 1]**

| Fat weight reducing effect on obesity model mice (KK-Ay mice) | | | |
|---|---|---|---|
| Compound (dose) | Compound A (1 mg/kg) | Compound B (3 mg/kg) | Fenofibrate (300 mg/kg) |
| Epididymal fat weight | 27% | - | 15% |
| Mesenteric fat weight | 20% | 17% | 13% |

| | | | |
|---|---|---|---|
| - : Not measured | | | |

## Claims

1. A compound selected from (R)-2-[3-[[N-(benzoxazol-2-yl)-N-3-(4-methoxyphenoxy)propyl]aminomethyl]phenoxy]butyric acid and (R)-2-[3-[[N-(5-fluorobenzoxazol-2-yl)-N-2-phenoxyethyl]aminomethyl]phenoxy]butyric acid, a salt thereof, or a solvate of the compound or the salt, for use in prevention and/or treatment of visceral fat obesity.

2. Pharmaceutical composition for use in a method of treatment and/or prevention of visceral fat obesity, the composition comprising a compound selected from (R)-2-[3-[[N-(benzoxazol-2-yl)-N-3-(4-methoxyphenoxy)propyl]aminomethyl]-phenoxy]butyric acid and (R)-2-[3-[[N-(5-fluorobenzoxazol-2-yl)-N-2-phenoxyethyl]aminomethyl]phenoxy]butyric acid, a salt thereof, or a solvate of the compound or the salt.

## Patentansprüche

1. Eine Verbindung, ausgewählt aus (R)-2-[3-[[N-(Benzoxazol-2-yl)-N-3-(4-methoxyphenoxy)propyl]aminomethyl]phenoxy]buttersäure und (R)-2-[3-[[N-(5-Fluorbenzoxazol-2-yl)-N-2-phenoxyethyl]aminomethyl]phenoxy]buttersäure, einem Salz davon oder einem Solvat der Verbindung oder des Salzes, zur Verwendung bei der Prävention und/oder Behandlung von viszeraler Adipositas.

2. Eine pharmazeutische Zusammensetzung zur Verwendung in einem Verfahren zur Behandlung und/oder Prävention von viszeraler Adipositas, wobei die Zusammensetzung eine Verbindung umfasst, ausgewählt aus (R)-2-[3-[[N-(Benzoxazol-2-yl)-N-3-(4-methoxyphenoxy)propyl]aminomethyl]phenoxy]buttersäure und (R)-2-[3-[[N-(5-Fluorbenzoxazol-2-yl)-N-2-phenoxyethyl]aminomethyl]phenoxy]buttersäure, einem Salz davon oder einem Solvat der Verbindung oder des Salzes.

## Revendications

1. Composé choisi parmi l'acide (R)-2-[3-[[N-(benzoxazol-2-yl)-N-3-(4-méthoxyphénoxy)propyl]amino-méthyl]phénoxy]butyrique et l'acide (R)-2-[3-[[N-(5-fluorobenzoxazol-2-yl)-N-2-phénoxyéthyl]aminométhyl]-phénoxy]butyrique, un sel de ceux-ci, ou un solvate du composé ou du sel, pour utilisation dans la prévention et/ou le traitement de l'obésité liée à la graisse viscérale.

2. Composition pharmaceutique pour utilisation dans une méthode de traitement et/ou de prévention de l'obésité liée à la graisse viscérale, la composition comprenant un composé choisi parmi l'acide (R)-2-[3-[[N-(benzoxazol-2-yl)-N-3-(4-méthoxyphénoxy)propyl]aminométhyl]phénoxy]-butyrique et l'acide (R)-2-[3-[[N-(5-fluorobenzoxazol-2-yl)-N-2-phénoxyéthyl]aminométhyl]phénoxy]butyrique, un sel de ceux-ci, ou un solvate du composé ou du sel.
